(19) Europäisches Patentamt
European Patent Office
Office européen des brevets

(11) Publication number: **0 049 506 A1**

# (12) EUROPEAN PATENT APPLICATION

(21) Application number: 81107934.2

(22) Date of filing: 05.10.81

(51) Int. Cl.³: C 07 C 103/52
A 61 K 37/02

(30) Priority: 06.10.80 US 194715
25.08.81 US 294864

(43) Date of publication of application:
14.04.82 Bulletin 82/15

(84) Designated Contracting States:
AT BE CH DE FR GB IT LI LU NL SE

(71) Applicant: MERCK & CO. INC.
126, East Lincoln Avenue P.O. Box 2000
Rahway, New Jersey 07065(US)

(72) Inventor: Harris, Elbert E.
220 Linden Avenue
Westfield New Jersey 07090(US)

(72) Inventor: Patchett, Arthur A.
1090 Minisink Way
Westfield New Jersey 07090(US)

(72) Inventor: Wu, Mu T.
36 Lance Drive
Clark New Jersey 07066(US)

(74) Representative: Abitz, Walter, Dr.-Ing. et al,
Abitz, Morf, Gritschneder P.O. Box 86 01 09
D-8000 München 86(DE)

## (54) N-carboxyalkyl dipeptides, a process for preparing and a pharmaceutical composition comprising the same.

(57) N-Carboxyalkyl dipeptides and derivatives thereof are disclosed which are useful as angiotensin converting enzyme inhibitors and as antihypertensives, and a process for preparing them.

Those compounds are represented by the formula

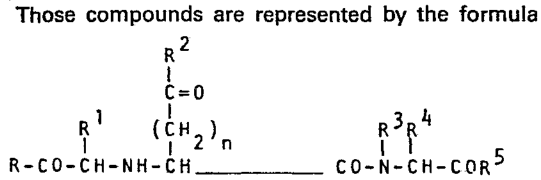

1

wherein

R and $R^5$ are individually hydroxy, lower alkoxy, aralkoxy;
$R^1$ is $C_{1-8}$ alkyl; substituted $C_{1-5}$ alkyl, substituted aralkyl or substituted heteroaralkyl;
$R^2$ is hydroxy, amino, alkoxy, aralkyloxy;
n is one, two or three;
$R^3$ is aryl, heteroaryl, cycloalkyl, aminoloweralkyl, aralkyl;
$R^4$ is hydrogen or loweralkyl; or
$R^3$ and $R^4$ are joined to form a ring having the following formulae:

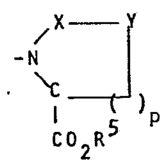

or

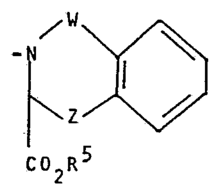

$-CH_2-S-$, $-CH_2CH-OR^6$, 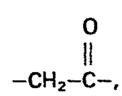, or

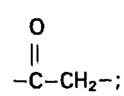;

$R^6$ is hydrogen or loweralkyl; p is 1, 2, or 3; W is a bond, $-CH_2-$, or C=O; and, Z is a bond, $-CH_2-$, or $-CH_2CH_2-$; and, the pharmaceutically acceptable salts thereof.

EP 0 049 506 A1

TITLE OF THE INVENTION

N- CARBOXYALKYL DIPEPTIDES, A PROCESS FOR PREPARING AND A PHARMACEUTICAL COMPOSITION COMPRISING THE SAME

BACKGROUND OF THE INVENTION

The invention in its broad aspects relates to N-carboxyalkyl dipeptides and derivatives thereof which are useful as angiotensin converting enzyme inhibitors and as antihypertensives. The compounds of this invention are represented by the following formula:

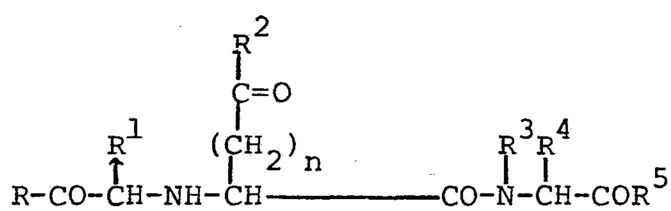

I

wherein

R and $R^5$ are individually hydroxy, lower alkoxy, aralkoxy;

$R^1$ is $C_{1-8}$ alkyl;
substituted $C_{1-5}$ alkyl in which the substituent is
- amino
- hydroxyl
- acylamino (such as acetylamino and benzoylamino)
- arylthio
- aryloxy
- aryl or
- heteroaryl; and

substituted aralkyl or substituted heteroaralkyl in which the substituent is
- halo or dihalo
- amino
- aminoloweralkyl
- hydroxy
- loweralkoxy, or
- loweralkyl;

$R^2$ is hydroxy, amino, alkoxy, aralkyloxy;
n is one, two or three
$R^3$ is aryl, heteroaryl, cycloalkyl, aminoloweralkyl, aralkyl;
$R^4$ is hydrogen or loweralkyl; or
$R^3$ and $R^4$ are joined to form a ring having the following formulae:

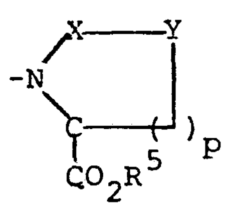

or

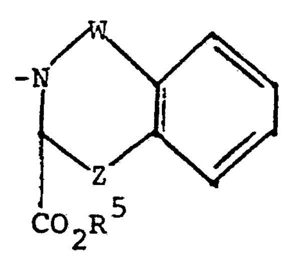

wherein X and Y taken together are $-CH_2CH_2-$,

$-CH_2-S-$, $-CH_2CH-OR^6$, $-CH_2-\overset{O}{\overset{\|}{C}}-$, or $-\overset{O}{\overset{\|}{C}}-CH_2-$

wherein $R^5$ is as defined above; $R^6$ is hydrogen or loweralkyl; p is 1, 2, or 3; W is a bond, $-CH_2-$, or C=O; and, Z is a bond, $-CH_2-$, or $-CH_2CH_2-$; and, the pharmaceutically acceptable salts thereof.

The loweralkyl groups, except where noted otherwise, represented by any of the variables include straight and branched chain hydrocarbon radicals from one to six carbon atoms, for example, methyl, ethyl, propyl, isopropyl, butyl, isobutyl, t-butyl, pentyl, isopentyl, or hexyl and the like. The aralkyl groups represented by any of the above variables have from one to three carbon atoms in the alkyl portion thereof and include for example, benzyl, phenethyl, and the like. Halo means chloro, bromo, iodo or fluoro. Aryl, where it appears in any of the radicals except where noted, represents phenyl, biphenyl, or naphthyl. Heteroaryl groups, where they appear, include for example, pyridyl, thienyl, furyl, indolyl, benzthienyl, imidazolyl and thiazolyl. Acylamino refers to loweralkanoylamino and aroylamino groups.

Preferred are those compounds of Formula I wherein:

R and $R^5$ are individually hydroxy, loweralkoxy, aralkoxy;

$R^1$ is substituted $C_{1-5}$ alkyl in which the substituent is amino, hydroxyl, acylamino, arylthio, aryloxy, aryl, or heteroaryl; and, substituted aralkyl or substituted heteroaralkyl in which the substituent is halo, amino, hydroxy, or loweralkoxy;

$R^2$ is hydroxy, alkoxy;

n is two or three; and,

$R^3$ and $R^4$ are joined to form a ring having the following formula:

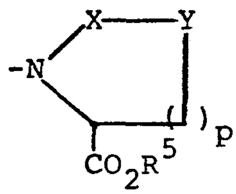

or

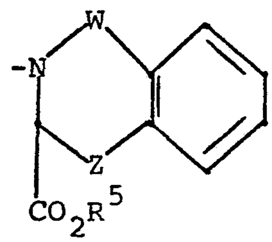

wherein X and Y taken together are $-CH_2CH_2-$, $-CH_2-S-$, $-CH_2CH-OR^6$; $R^6$ is hydrogen, loweralkyl; p is 1 or 2; W is a bond, or $-CH_2-$; and, Z is a bond, $-CH_2-$, or $-CH_2CH_2-$.

Most preferred are compounds of Formula I wherein:

R and $R^5$ are individually hydroxy, loweralkoxy, aralkoxy;

$R^1$ is aralkyl or heteroaralkyl wherein the alkyl portions contain 1-3 carbon atoms and the aryl and heteroaryl groups may be substituted by halo or hydroxy;

$R^2$ is hydroxy, alkoxy;

n is two or three; and,

$R^3$ and $R^4$ are joined to form a ring having the following formula:

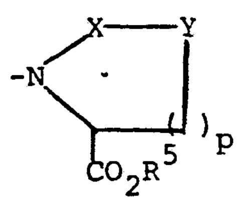

or

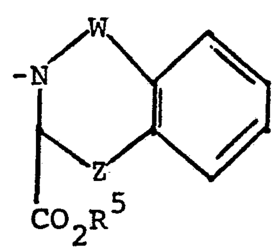

wherein X and Y taken together are $-CH_2CH_2-$, $-CH_2-S-$, $-CH_2CH-OR^6$; $R^6$ is hydrogen, loweralkyl; p is 1; W is $-CH_2-$; and, Z is $-CH_2-$.

The preferred and most preferred compounds of Formula I also include the pharmaceutically acceptable salts thereof.

The products of Formula I and the preferred subgroups can be produced by the following methods.

Method I, Route 1:

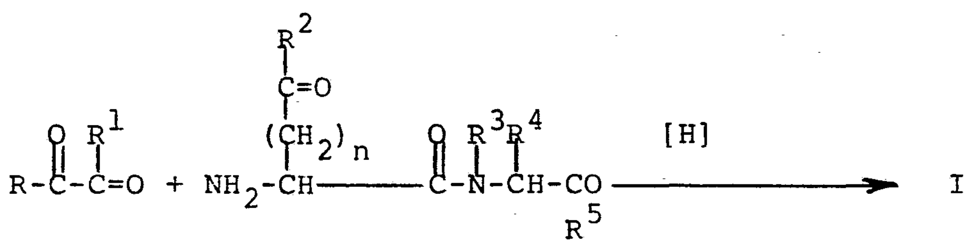


II III

Keto acid (or ester) II is condensed with dipeptide III in aqueous solution, optimally near neutrality, or in suitable organic solvent ($CH_3CN$ for example) in the presence of sodium cyanoborohydride to give I. Alternatively, the intermediate Schiff base or enamine base may be catalytically reduced to yield product I, for example, by hydrogen in the presence of 10% palladium on carbon or of Raney nickel. The ratio of diasteriomeric products formed may be altered by choice of catalyst.

When R and $R^5$ are alkoxy or aralkoxy, they can be removed by hydrolysis to give I where R and $R^5$ are OH. However, these functions often confer pharmacological advantages to these drugs.

Alternatively II can be condensed with an amino acid IV (R and $R^2 \neq$ OH).

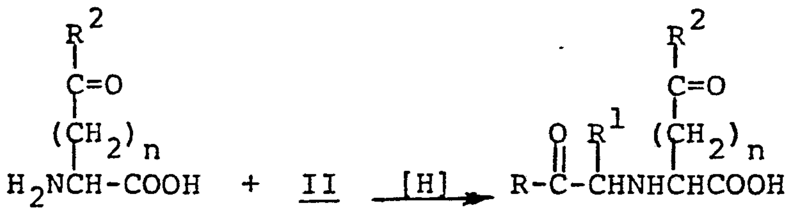


IV V

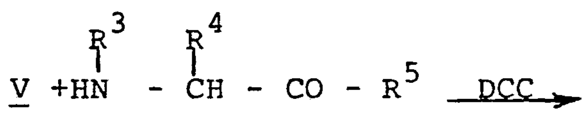


(DCC = Dicyclohexylcarbodiimide)
or
DPPA

(VI)

(DPPA = Diphenylphosphoryl azide)

under the same conditions to yield substituted amino acid V. Subsequent coupling by known methods with amino acid derivative VI gives I.

The above methods encompass reactive group protection during the coupling reaction, for example, by N-formyl, N-t-butoxycarbonyl and N-carbobenzyloxy groups followed by their removal to yield I. Furthermore, the R function may include removable ester groups such as benzyl, ethyl, or t-butyl. Condensing agents in this synthesis are typically those useful in peptide chemistry such as dicyclohexylcarbodiimide (DCC) or diphenylphosphoryl azide (DPPA) or V may be activated via the intermediacy of active esters such as that derived from 1-hydroxybenzotriazole. Also, synthesis of dipeptides III and dipeptide analogs IV are achieved by methods standard in peptide chemistry

from starting materials which are known or which can be made by known methods from known starting materials.

In products of general Formula I, the carbon atoms to which $R^1$, $R^2$-CO-$(CH_2)_n$- and $R^4$ are attached are asymmetric when $R^1$ and $R^4$ are not hydrogens. The compounds exist in diastereoisomeric forms or in mixtures thereof. The above described synthesis can utilize racemates, enantiomers or diastereomers as intermediates or as starting materials. When diastereomeric products result, they can be separted by conventional chromatographic or fractional crystallization methods. In general, the aminoacid part-structures, i.e.,

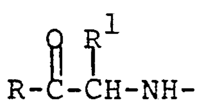 , 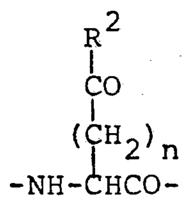 , and 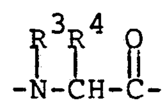

of Formula I are preferred in the S-configuration.

The compounds of this invention form salts with various inorganic and organic acids and bases which are also within the scope of the invention. Such salts include ammonium sats, alkali metal salts like sodium and potassium salts, alkaline earth metal salts like the calcium and magnesium salts, salts with organic bases e.g., dicyclohexylamine salts, N-methyl-D-glucamine, salts with amino acids like arginine, lysine and the like. Also, salts with organic and inorganic acids may be prepared, e.g., HCl, HBr, $H_2SO_4$, $H_3PO_4$, methanesulfonic, toluensulfonic, maleic, fumaric, camphorsulfonic.

Non-toxic physiologically acceptable salts are preferred, although other salts are also useful, e.g., in isolating or purifying the product.

The salts may be formed by conventional means, as by reacting the free acid or free base forms of the product with one or more equivalents of the appropriate base or acid in a solvent or medium in which the salt is insoluble, or in a solvent such as water which is then removed *in vacuo* or by freeze-drying or by exchanging the cations of an existing salt for another cation on a suitable ion exchange resin.

The compounds of this invention inhibit angiotensin converting enzyme and thus block conversion of the decapeptide angiotensin I to angiotensin II. Angiotensin II is a potent pressor substance. Thus, blood-pressure lowering can result from inhibition of its biosynthesis especially in animals and humans whose hypertension is angiotensin II related. Furthermore, converting enzyme degrades the vasodepressor substance, bradykinin. Therefore, inhibitors of angiotensin converting enzyme may also lower blood-pressure by potentiation of bradykinin. Although the relative importance of these and other possible mechanisms remains to be established, inhibitors of angiotensin converting enzyme are effective antihypertensive agents in a variety of animal models and are useful clinically, for example, in many human patients with renovascular, malignant and essential hypertension. See, for example, A. B. Atkinson and J. I. S. Robertson, *Lancet* ii, 836 (1979) and references therein.

The evaluation of converting enzyme inhibitors is guided by in vitro enzyme inhibition assays. For example, a useful method is that of Y. Piquilloud, A. Reinharz and M. Roth, Biochem. Biophys. Acta, 206, 136 (1970) in which the hydrolysis of carbobenzyloxyphenylalanylhistidinylleucine is measured. In vitro evaluations may be made, for example, in normotensive rats challenged with angiotensin I by the technique of J. R. Weeks and J. A. Jones, Proc. Soc. Exp. Biol. Med., 104, 646 (1960) or in a high renin rat model such as that of S. Koletsky et. al., Proc. Soc. Exp. Biol. Med., 125, 96 (1967).

Thus, the compounds of this invention are useful as hypertensives in treating hypertensive mammals, including humans, and they can be utilized to achieve the reduction of blood pressure by formulating in compositions such as tablets, capsules or elixirs for oral administration or in sterile solutions or suspensions for parenteral administration. The compounds of this invention can be administered to patients in need of such treatment in dosages that will at least be pharmaceutically active. Thus, although the dose will vary depending on severity of disease, weight of patient and other factors which a person skilled in the art will recognize, the dosage range will be about 10 to 500 mg per patient which can be given several times a day.

Also, the compounds of this invention may be given in combination with diuretics or other antihypertensives. Typically these are combinations whose individual per day dosages range from one fifth of the minimal recommended clinical dosages to the

maximum recommended levels for the entities when they are given singly. To illustrate these combinations, one of the antihypertensives of this invention effective clinically in the range 50-500 milligrams per day can be effectively combined at levels ranging from 10-500 milligrams per day with the following antihypertensives and diuretics in dose ranges per day as indicated:

hydrochlorothiazide (10-100 mg), timolol (5-60 mg), methyl dopa (65-2000 mg), the pivaloyloxyethyl ester of methyl dopa (30-1000 mg), indacrinone and variable ratios of its enantiomers (25-150 mg) and (+)-4-{3-{-[2-(1-hydroxycyclohexyl)ethyl]-4-oxo-2-thiazolidinyl}-propyl}benzoic acid (10-100 mg).

In addition, the triple drug combinations of hydrochlorothiazide (10-100 mg) plus amiloride (5-20 mg) plus converting enzyme inhibitor of this invention (10-500 mg) or hydrochlorothiazide (10-100 mg) plus timolol (5-50 mg) plus the converting enzyme inhibitor of this invention (10-500 mg) are effective combinations to control blood pressure in hypertensive patients.

The above dose ranges will be adjusted on a unit basis as necessary to permit divided daily dosage.

Typically the compounds and combinations shown above are formulated into pharmaceutical compositions as discussed below.

Accordingly, about 10 to 500 mg of a compound or mixture of compounds of Formula I or a physiologically acceptable salt is compounded with a physiologically acceptable vehicle, carrier, excipient,

binder, preservative, stabilizer, flavor, etc., in a unit dosage form as called for by accepted pharmaceutical practice. The amount of active substance in these compositions or preparations is such that a suitable dosage in the range indicated is obtained.

Illustrative of the adjuvants which may be incorporated in tablets, capsules and the like are the following: a binder such as gum tragacanth, acacia, corn starch or gelatin; an excipient such as microcrystalline cellulose; a disintegrating agent such as corn starch, pregelatinized starch, alginic acid and the like; a lubricant such as magnesium stearate; a sweetening agent such as sucrose, lactose or saccharin; a flavoring agent such as peppermint, oil of wintergreen or cherry. When the dosage unit form is a capsule, it may contain, in addition to materials of the above type, a liquid carrier such as fatty oil. Various other materials may be present as coatings or to otherwise modify the physical form of the dosage unit. For instance, tablets may be coated with shellac, sugar or both. A syrup or elixir may contain the active compound, sucrose as a sweetening agent, methyl and propyl parabens as preservatives, a dye and a flavoring such as cherry or orange flavor.

Sterile compositions for injection can be formulated according to conventional pharmaceutical practice by dissolving or suspending the active substance in a vehicle such as water for injection, a naturally occurring vegetable oil like sesame oil, coconut oil, peanut oil, cottonseed oil, etc. or a

synthetic fatty vehicle like ethyl oleate or the like. Buffers, preservatives, antioxidants and the like can be incorporated as required.

The following examples are illustrative of the invention and constitute especially preferred embodiments. As mentioned earlier, the preferred diastereomers of these examples can be isolated by column chromatography or fractional crystallization.

EXAMPLE 1

N-(1-Carboxy-3-phenylpropyl)-L-α-glutamyl-L-Proline

The dipeptide L-α-glutamyl-L-proline is prepared by DCC condensation of N-CBZ-L-glutamic acid-γ-t-butyl ester and L-proline-t-butyl ester followed by hydrogenolysis of CBZ and TFA hydrolysis of the t-butyl esters.

An aqueous suspension of 2-oxo-4-phenyl-butyric acid (1.74 g) and L-α-glutamyl-L-proline TFA salt (700 mg) is adjusted to pH 7 with sodium hydroxide then treated with sodium cyanoborohydride (368 mg) at room temperature for several days. The product is adsorbed on strong acid ion exchange resin and eluted with 2% pyridine in water. Product-rich cuts are stripped to a glass which is freeze-dried from a mixture of dioxane and water to a white fluffy solid. Crude product is purified by LH-20 gel filtration chromatography in methanol. The mass spectrum shows a molecular ion at 622 m/e for the trisilylated species. The nmr spectrum is consistent with structure.

EXAMPLE 2

N-(1-Ethoxycarbonyl-3-phenylpropyl)-L-α-glutamyl-L-proline

Powdered 4A molecular sieves (1 g) are added to a solution of L-α-glutamyl-L-proline (1 mmol) and ethyl 2-oxo-4-phenylbutyrate (5 mmol) in ethanol. Sodium cyanoborohydride (3 mmol in ethanol) is added portionwise to the suspension. The reaction is stirred at room temperature for several days. N-(1-ethoxycarbonyl-3-phenylpropyl)-L-α-glutamyl-L-proline is isolated by chromatography.

EXAMPLE 3

1-[2-[(1-Carboxy-3-phenylpropyl)amino]-4-(ethoxycarbonyl)-L-butyryl]-L-proline

A suspension of 2-oxo-4-phenylbutyric acid (5 mmol) in water is adjusted to pH 7 with dilute sodium hydroxide and freeze dried. The residue is dissolved in ethanol and treated with L-2-amino-4-ethoxycarbonylbutyryl-L-proline (1 mmol) and powdered 4A molecular sieves (1 g). Sodium cyanoborohydride (3 mmol in ethanol) is added portionwise to the stirred suspension. 1-[2-[(1-carboxy-3-phenylpropyl)amino-4-ethoxycarbonyl-L-butyryl]-L-proline is isolated from the reaction mixture by chromatography.

EXAMPLE 4

1-[2-[1-Ethoxycarbonyl-3-phenylpropyl)amino]-4-(ethoxycarbonyl)-L-butyryl]-L-proline

In the manner described in Example 2, ethyl 2-oxo-4-phenylbutyrate and L-2-amino-4-carboethoxy-

butyryl proline are condensed to afford 1-[2-[1-ethoxycarbonyl-3-phenylpropyl)amino]-4-(ethoxycarbonyl)-L-butyryl]-L-proline.

EXAMPLE 5

N-(1-Carboxy-3-phenylpropyl)-L-α-glutamyl-L-proline diethyl ester

In the manner described in Example 3, 2-oxo-4-phenylbutyric acid and L-α-glutamyl-L-proline diethyl ester are condensed to yield N-1-carboxy-3-phenylpropyl)-L-α-glutamyl-L-proline diethyl ester.

EXAMPLE 6

N-(1-Ethoxycarbonyl-3-phenylpropyl)-L-α-glutamyl-L-proline diethyl ester

Condensation of ethyl 2-oxo-4-phenylbutyrate and L-α-glutamyl-L-proline diethyl ester in the manner described in Example 2, affords N-(1-ethoxycarbonyl-3-phenylpropyl)-L-α-glutamyl-L-proline diethyl ester.

EXAMPLE 7

N-[1-Carboxy-3-(3-indolyl)propyl]-L-α-glutamyl-L-proline

In the manner described in Example 1, 2-oxo-4-(3-indolyl)butyric acid and L-α-glutamyl-L-proline are condensed in the presence of sodium cyanoborohydride to yield N-[1-carboxy-3-(3-indolyl)propyl]-L-α-glutamyl-L-proline.

EXAMPLE 8

N-[1-Ethoxycarbonyl-3-(3-indolyl)propyl]-L-α-glutamyl-L-proline

Condensation of ethyl 2-oxo-4-(3-indolyl)-butyrate and L-α-glutamyl-L-proline in the manner

described in Example 2 affords N-[1-ethoxycarbonyl-3-(3-indolyl)propyl]-L-α-glutamyl-L-proline.

EXAMPLE 9

1-[2-[[1-Carboxy-3-(1H-indol-3-yl)propyl]amino]-4-(ethoxy-carbonyl)-L-butyryl]-L-proline

In the manner described in Example 3, 2-oxo-4-(3-indolyl)butyric acid and L-2-amino-4-carboethoxy-L-butyryl-L-proline are condensed in the presence of sodium cyanoborohydride to yield 1-[2[[1-carboxy-3-(1H-indol-3-yl)propyl]amino]-4-(ethoxycarbonyl)-L-butyryl]-L-proline.

EXAMPLE 10

1-[2-[[1-Ethoxycarbonyl-3-(1H-indol-3-yl)propyl]amino]-4-(ethoxycarbonyl)-L-butyryl]-L-proline

Condensation of ethyl 2-oxo-4(3-indolyl)-butyrate and L-2-amino-4-carboethoxy-L-butyryl-L-proline in the manner described in Example 2 affords 1-[2-[[1-ethoxycarbonyl-3-(1-indol-3-yl)propyl]amino]-4-(ethoxy-carbonyl)-L-butyryl]-L-proline.

EXAMPLE 11

N-[1-Carboxy-3-(p-chlorophenyl)propyl]-L-α-glutamyl-L-proline

In the manner described in Example 1, 2-oxo-4-(p-chlorophenyl)butyric acid and L-α-glutamyl-L-proline are condensed to afford N-[1-carboxy-3-(p-chlorophenyl)propyl]-L-α-glutamyl-L-proline.

EXAMPLE 12

1-[2-[1-Carboxy3-(p-chlorophenyl)propyl]amino]-4-(ethoxy-carbonyl)-L-butyryl]-L-proline

Condensation of 2-oxo-4(p-chlorohenyl)butyric acid and L-2-amino-4-carboethoxy-L-butyryl-L-proline in the manner described in Example 3, affords 1-[2-[1-carboxy-3-(p-chlorophenyl)propyl]amino]-4-(ethoxy-carbonyl)-L-butyryl]-L-proline.

EXAMPLE 13

N-(1-Carboxy-5-aminopentyl)-L-α-glutamyl-L-proline

In the manner described in Example 3, 2-oxo-6-phthalimidohexanoic acid and L-α-glutamyl-L-proline are condensed in the presence of sodium cyanoborohydride. Following hydrazinolysis of the phthalimido protecting group the desired product, N-(1-carboxy-5-aminopentyl)-L-α-glutamyl-L-proline is obtained.

EXAMPLE 14

1-{2-[1-Carboxy-5-aminopentyl)amino]-4-(ethoxycarbonyl)-L-butyryl}-L-proline

In the manner described i Example 3, 2-oxo-6-phthalimidohexanoic acid and L-2-amino-4-carboethoxy-butyryl-L-proline are condensed in the presence of sodium cyanoborohydride. The phthalimido protecting group is removed by hydrazinolysis to afford 1-[2-[(1-carboxy-5-amino-pentyl)amino]-4-(ethoxycarbonyl)-L-butyryl]-L-proline.

EXAMPLE 15

N-(1-Carboxy-3-phenylpropyl)-L-glutaminyl-L-proline

Condensation of 2-oxo-4-phenylbutyric acid and L-glutaminyl-L-proline as described in Example 15 affords N-(1-carboxy-3-phenylpropyl)-L-glutaminyl-L-proline.

EXAMPLE 16

N-(1-Carboxy-3-phenylpropyl)-L-asparaginyl-L-proline

In the manner described in Example 15, 2-oxo-4-phenylbutyric acid and L-asparaginyl-L-proline are condensed in the presence of sodium cyanoborohydride to yield N-(1-carboxy-3-phenylpropyl)-L-asparaginyl-L-proline. The mass spectrum shows a molecular ion at 607 m/e for the trisilyated species. The nmr spectrum is consistent with structure.

EXAMPLE 17

N-(1-Carboxy-3-phenylpropyl)-L-α-glutamyl-L-4-thiazolidine carboxylic acid

In the manner described in Example 15, 2-oxo-4-phenylbutyric acid and L-α-glutamyl-L-4-thiazolidine carboxylic acid are condensed to afford N-(1-carboxy-3-phenylpropyl)-L-α-glutamyl-L-4-thiazolidine carboxylic acid.

EXAMPLE 18

N-(1-Ethoxycarbonyl-3-phenylpropyl)-L-α-glutamyl-L-4-thiazolidine carboxylic acid

Condensation of 2-oxo-4-phenylbutyrate and L-α-glutamyl-L-4-thiazolidine carboxylic acid in the manner

described in Example 16 affords N-(1-ethoxycarbonyl-3-phenylpropyl)-L-α-glutamyl-L-4-thiazolidine carboxylic acid.

EXAMPLE 19

1-{2-[(1-Carboxy-3-phenylpropyl)amino]-5-carboxy-L-norvalyl}-L-proline

In the manner described in Example 15, 2-oxo-4-phenylbutyric acid and L- -amino-adipyl-L-proline are condensed to yield 1-{2-[(1-carboxy-3-phenylpropyl)-amino-5-carboxy-L-norvalyl}-L-proline.

EXAMPLE 20

1-{2-[(1-Ethoxycarbonyl-3-phenylpropyl)amino]-5-carboxy-L-norvalyl}-L-proline

In the manner described in Example 16, ethyl-2-oxo-4-phenylbutyrate and L-α-amino-adipoyl-L-proline are condensed in the presence of sodium cyanoborohydride. Following isolation by chromatography 1-{2-[(1-ethoxycarbonyl-3-phenylpropyl)amino]-5-carboxy-L-norvalyl}-L-proline is obtained.

EXAMPLE 21

1-{2-[(1-Carboxy-3-phenylpropyl)amino]-5-(ethoxy-carbonyl)-L-norvalyl}-L-proline

Condensation of 2-oxo-4-phenylbutyric acid with L-2-amino-5-carboethoxy-norvalyl-L-proline in Example 17 affords 1-{2-[(1-carboxy-3-phenylpropyl)-amino]-5-(ethoxycarboyl)-L-norvalyl}-L-proline.

EXAMPLE 22

1-{2-[(1-Ethoxycarbonyl-3-phenylpropyl)amino]-5-(ethoxy-carbonyl)-L-norvalyl}-L-proline

In the manner described in Example 16 ethyl 2-oxo-4-phenylbutyrate and L-2-amino-5-carboethoxy-norvalyl-L-proline are condensed to afford 1- 2-[(1-ethoxycarbonyl-3-phenylpropyl)amino]-5-(ethoxycarbonyl)-L-norvalyl -L-proline.

EXAMPLE 23

Additional Dipeptide Derivatives of Formula I

Aminoacids and their alkyl and aralkyl esters selected from Table I can be coupled with L-glutamic acid, L-aspartic acid, and L-α-aminoadipic acid as their ω-alkyl or ω-aralkyl esters using appropriate N-protecting groups as illustrated, but not limited to those disclosed, in Example 1. The resulting deblocked dipeptides can be reductively condensed with α-ketoacids and α-ketoesters listed in Table II and elsewhere in the Examples herein by the method disclosed in Example 1. The products which can be obtained, after optional removal of protecting groups, and which are within the scope of this invention are summarized in Table III.

TABLE I

Additional Aminoacids Contributing to the 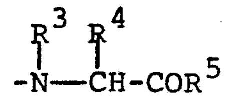 Unit in Formula I

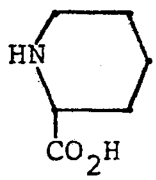

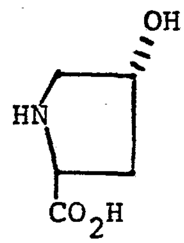

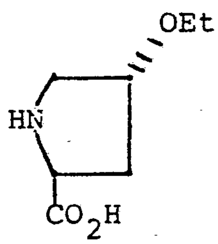

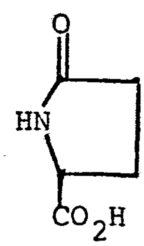

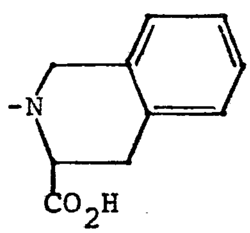

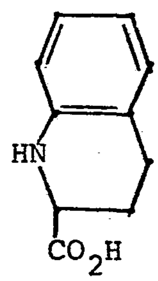

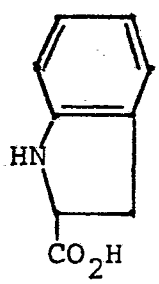

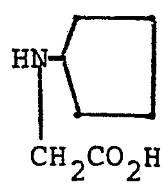

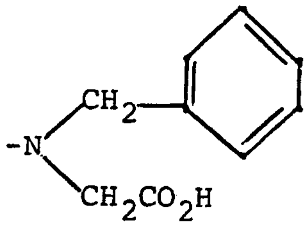 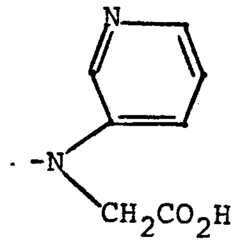

TABLE II

Additional α-Ketoacids and α-Ketoesters Providing $R^1$-CH-COR Units in Formula I

(R=OH, -OEt, $-OCH_2$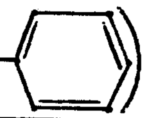)

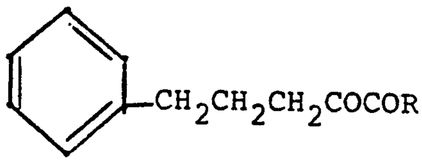 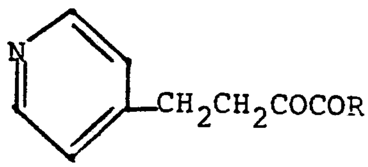

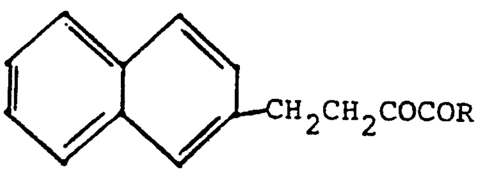 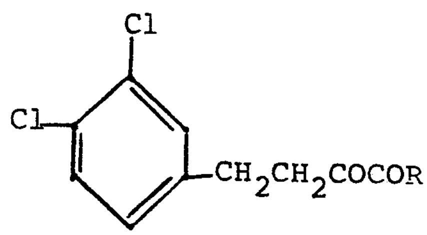

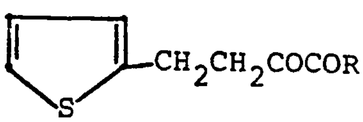 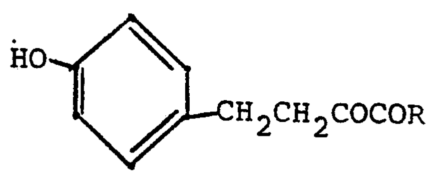

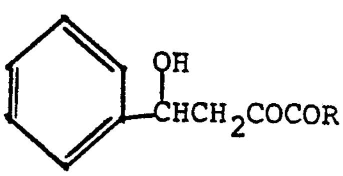 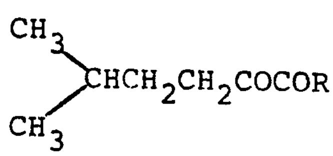

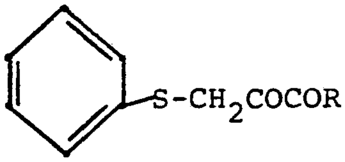

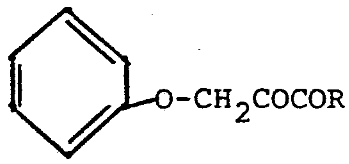

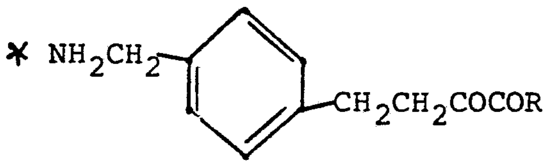

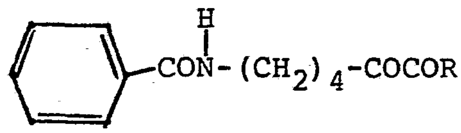

* $NH_2$ protected as t-BOC

TABLE III

Additional Compounds of Formula I
(R, $R^2$ and $R^5$ = -OH, -OEt, -$OCH_2$ 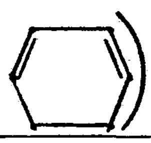)

| $R^1$ | n | 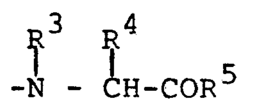 |
|---|---|---|
| 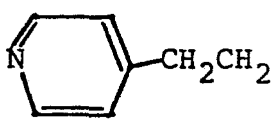 | 3 | 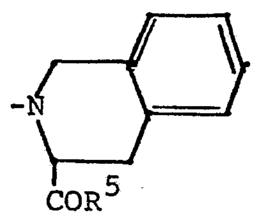 |
| 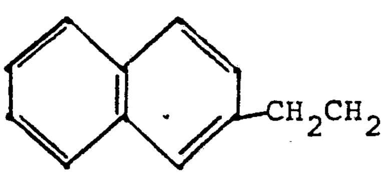 | 2 | 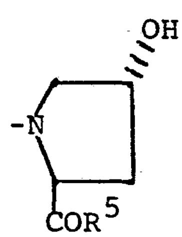 |

| | | |
|---|---|---|
| 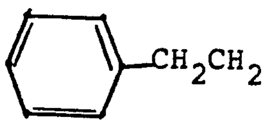 | 2 | 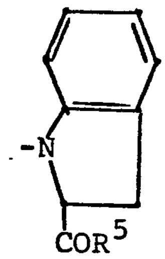 |
| 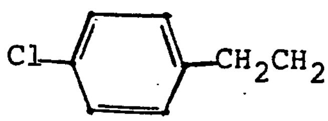 | 3 | 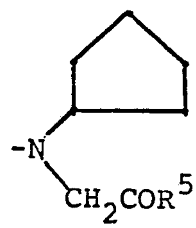 |
| 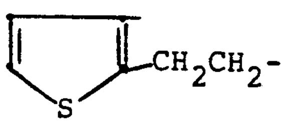 | 1 | 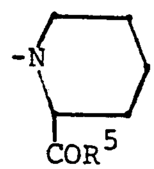 |
| 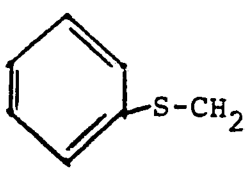 | 2 | 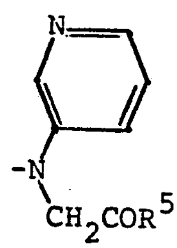 |
| 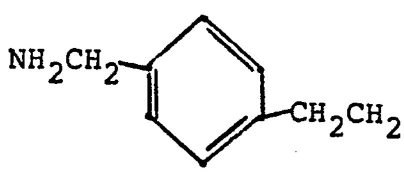 | 3 | 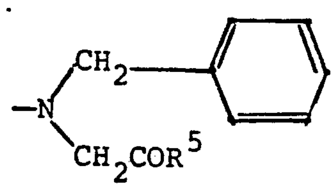 |

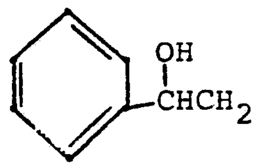

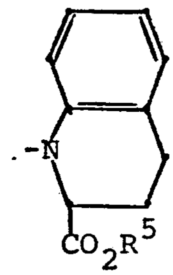

WHAT IS CLAIMED IS:

1. A compound of the formula:

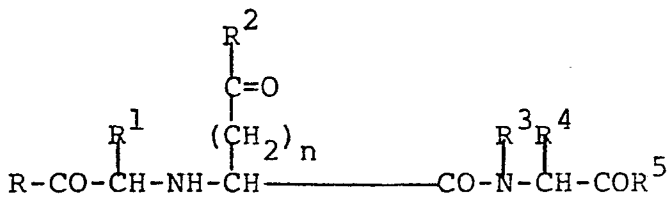

I

wherein

R and $R^5$ are individually hydroxy, lower alkoxy, aralkoxy;

$R^1$ is $C_{1-8}$ alkyl;

substituted $C_{1-5}$ alkyl in which the substituent is

amino
hydroxyl
acylamino
arylthio
aryloxy
aryl or heteroaryl, and

substituted aralkyl or substituted heteroaralkyl in which the substituent is

halo or dihalo
amino
aminoloweralkyl
hydroxy
loweralkoxy, or
loweralkyl;

$R^2$ is hydroxy, amino, alkoxy, aralkyloxy;

n is one, two or three;

$R^3$ is aryl, heteroaryl, cycloalkyl, aminoloweralkyl, aralkyl;
$R^4$ is hydrogen or loweralkyl; or
$R^3$ and $R^4$ are joined to form a ring having the following formulae:

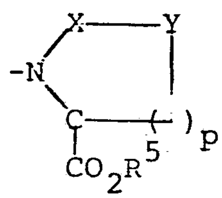

or

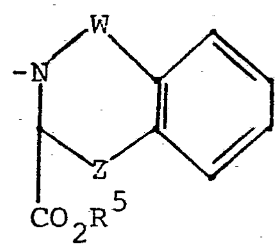

wherein X and Y taken together are $-CH_2CH_2-$, $-CH_2-S-$, $-CH_2CH-OR^6$, $-CH_2-\overset{O}{\overset{\|}{C}}-$, or $-\overset{O}{\overset{\|}{C}}-CH_2-$, wherein $R^5$ is as defined above; $R^6$ is hydrogen or loweralkyl; p is 1, 2, or 3; W is a bond, $-CH_2-$, or C=O; and, Z is a bond, $-CH_2-$, or $-CH_2CH_2-$; and, the pharmaceutically acceptable salts thereof.

2. A compound of Claim 1 which is a member of the group:
N-(1-carboxy-3-phenylpropyl)-L-α-glutamyl-L-proline;
N-(1-ethoxycarbonyl-3-phenylpropyl)-L-α-glutamyl-L-proline;
N-(1(S)-carboxy-3-phenylpropyl)-L-α-glutamyl-L-proline.
N-(1(S)-ethoxycarbonyl-3-phenylpropyl)-L-α-glutamyl-L-proline.

3. A pharmaceutical composition useful in the treatment of hypertension which comprises a pharmaceutically acceptable carrier; and, an

antihypertensively effective amount of a compound having the formula:

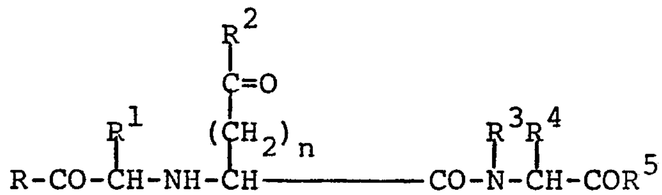

I

wherein

R and $R^5$ are individually hydroxy, lower alkoxy, aralkoxy;

$R^1$ is $C_{1-8}$ alkyl;

substituted $C_{1-5}$ alkyl in which the substituent is

amino,

hydroxyl,

acylamino,

arylthio,

aryloxy,

aryl or heteroaryl, and

substituted aralkyl or substituted heteroaralkyl in which the substituent is

halo or dihalo

amino

aminoloweralkyl

hydroxy

loweralkoxy, or

loweralkyl;

$R^2$ is hydroxy, amino, alkoxy, aralkyloxy;

n is one, two or three;

$R^3$ is aryl, heteroaryl, cycloalkyl, aminoloweralkyl, aralkyl;

$R^4$ is hydrogen or loweralkyl; or,

$R^3$ and $R^4$ are joined to form a ring having the following formulae:

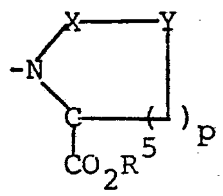

or

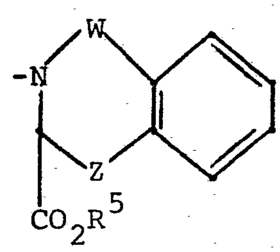

wherein X and Y taken together are $-CH_2CH_2-$, $-CH_2-S-$, $-CH_2CH-OR^6$, $-CH_2-\overset{O}{\overset{\|}{C}}-$, or $-\overset{O}{\overset{\|}{C}}-CH_2-$ wherein $R^5$ is as defined above; $R^6$ is hydrogen or loweralkyl; p is 1, 2, or 3; W is a bond, $-CH_2-$, or C=O; Z is a bond, $-CH_2-$, or $-CH_2CH_2-$; and, the pharmaceutically acceptable salts thereof.

4. The composition of Claim 3 wherein said compound is a member of the group:

N-(1-carboxy-3-phenylpropyl)-L-α-glutamyl-L-proline;

N-(1-ethoxycarbonyl-3-phenylpropyl)-L-α-glutamyl-L-proline;

N-(1(S)-carboxy-3-phenylpropyl)-L-α-glutamyl-L-proline; and,

N-(1(S)-ethoxycarbonyl-3-phenylpropyl)-L-α-glutamyl-L-proline.

5. The composition of Claim 3 which includes an antihypertensive and/or diuretic compound selected from the group consisting of amiloride, hydrochlorothiazide, methyldopa, the pilvaloyloxyethyl ester of methyldopa, indacrinone and variable ratios of

its enantiomers, (+)-4-{3-{[2-(1-hydroxycyclohexyl)-ethyl]-4-oxo-2-thiazolidinyl}propyl}-benzoic acid, timolol, as well as admixtures and combinations thereof.

6. A process for preparing a compound of the formula:

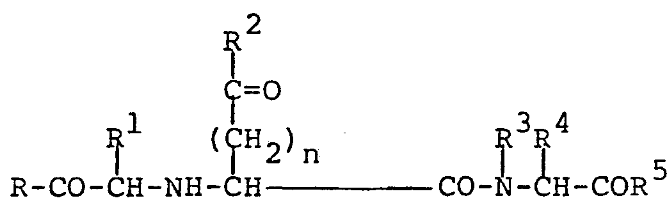

I

wherein

R and $R^5$ are individually hydroxy, lower alkoxy, aralkoxy;

$R^1$ is $C_{1-8}$ alkyl;

substituted $C_{1-5}$ alkyl in which the substituent is

amino,

hydroxyl,

acylamino,

arylthio,

aryloxy,

aryl or heteroaryl, and

substituted aralkyl or substituted heteroaralkyl in which the substituent is

halo or dihalo

amino

aminoloweralkyl

hydroxy

loweralkoxy, or

loweralkyl;

$R^2$ is hydroxy, amino, alkoxy, aralkyloxy;

n is one, two or three;

$R^3$ is aryl, heteroaryl, cycloalkyl, aminoloweralkyl, aralkyl;

$R^4$ is hydrogen or loweralkyl; or,

$R^3$ and $R^4$ are joined to form a ring having the following formulae:

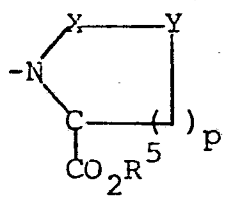

or

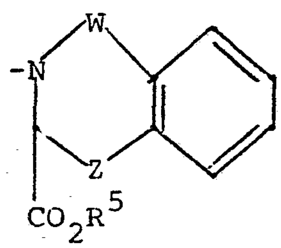

wherein X and Y taken together are $-CH_2CH_2-$, $-CH_2-S-$, $-CH_2CH-OR^6$, 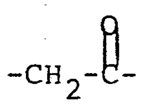, or 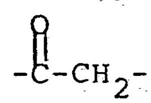 wherein $R^5$ is as defined above; $R^6$ is hydrogen or loweralkyl; p is 1, 2, or 3; W is a bond, $-CH_2-$, or C=O; and, Z is a bond, $-CH_2-$, or $-CH_2CH_2-$; and, the pharmaceutically acceptable salts thereof, which process comprises reacting a ketone of the formula:

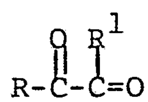

wherein R and $R^1$ are the same as defined above and may include suitable protection of any reactive groups, with a dipeptide or protected dipeptide of the formula:

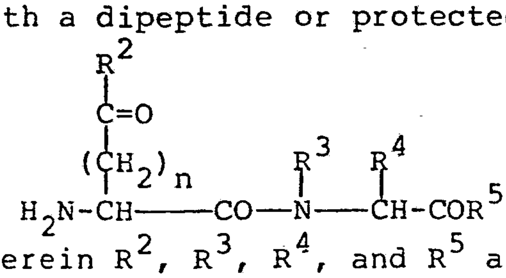

wherein $R^2$, $R^3$, $R^4$, and $R^5$ are as defined above and $R^2$, $R^4$, and $R^5$ may include suitable protection of reactive groups, in the presence of a suitable reducing agent, followed by removal of the

protecting groups, if necessary, to yield the desired product and, if desired, isolating the biologically more active isomer by chromatography or fractional crystallization and, if further desired, preparing a salt of the product by conventional means.

## CLAIMS FOR AUSTRIA

1. A process for preparing a compound of the formula:

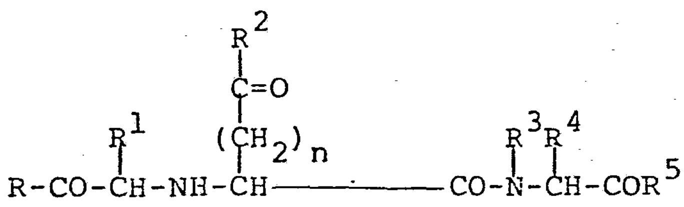

I

wherein

R and $R^5$ are individually hydroxy, lower alkoxy, aralkoxy;

$R^1$ is $C_{1-8}$ alkyl;

substituted $C_{1-5}$ alkyl in which the substituent is

amino,

hydroxyl,

acylamino,

arylthio,

aryloxy,

aryl or heteroaryl, and

substituted aralkyl or substituted heteroaralkyl in which the substituent is

halo or dihalo

amino

aminoloweralkyl

hydroxy

loweralkoxy, or

loweralkyl;

$R^2$ is hydroxy, amino, alkoxy, aralkyloxy;

n is one, two or three;

$R^3$ is aryl, heteroaryl, cycloalkyl, aminoloweralkyl, aralkyl;

$R^4$ is hydrogen or loweralkyl; or,

$R^3$ and $R^4$ are joined to form a ring having the following formulae:

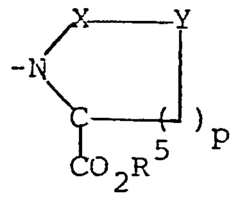

or

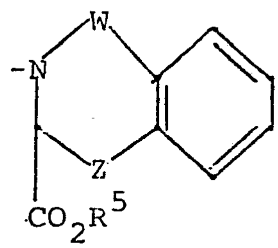

wherein X and Y taken together are $-CH_2CH_2-$, $-CH_2-S-$, $-CH_2CH-OR^6$, $-CH_2-\overset{O}{\overset{\|}{C}}-$, or $-\overset{O}{\overset{\|}{C}}-CH_2-$ wherein $R^5$ is as defined above; $R^6$ is hydrogen or loweralkyl; p is 1, 2, or 3; W is a bond, $-CH_2-$, or C=O; and, Z is a bond, $-CH_2-$, or $-CH_2CH_2-$; and the pharmaceutically acceptable salts thereof, which process comprises

a) reacting a ketone of the formula

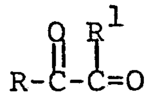

II

wherein R and $R^1$ are the same as defined above and may include suitable protection of any reactive groups, with a dipeptide or protected dipeptide of the formula:

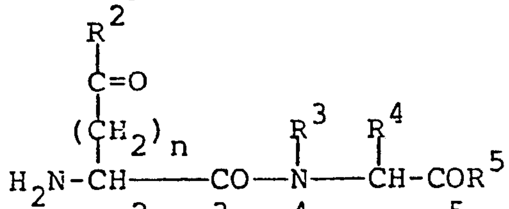

wherein $R^2$, $R^3$, $R^4$, and $R^5$ are as defined above and $R^2$, $R^4$, and $R^5$ may include suitable protection of reactive groups, in the presence of a suitable reducing agent, followed by removal of the

protecting groups, if necessary, to yield the desired product, or

b) condensing the above ketone II with an amino acid IV (R and $R^2 \neq$ OH)

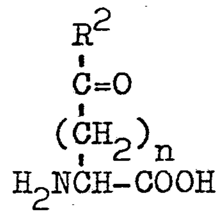

IV

under the same conditions as describes above to yield substituted amino acid V

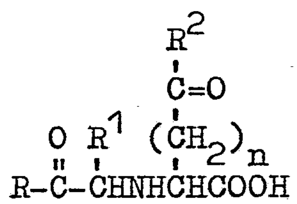

V

and subsequent coupling by known methods with amino acid derivative VI

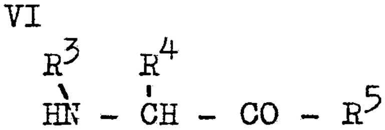

to give the desired product and,
if desired, isolating the biologically more active isomer by chromatography or fractional crystallization and, if further desired, preparing a salt of the product by conventional means.

2. A process according to Claim 1 for preparing a compound which is a member of the group:

N-(1-carboxy-3-phenylpropyl)-L-α-glutamyl-L-proline;
N-(1-ethoxycarbonyl-3-phenylpropyl)-L-α-glutamyl-L-proline;
N-(1(S)-carboxy-3-phenylpropyl)-L-α-glutamyl-L-proline.
N-(1(S)-ethoxycarbonyl-3-phenylpropyl)-L-α-glutamyl-L-proline.

0049506

European Patent Office

# EUROPEAN SEARCH REPORT

Application number

EP 81 10 7934

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | EP - A - 0 012 401 (MERCK & CO)<br>* Whole document *<br>---- | 1-6 | C 07 C 103/52<br>A 61 K 37/02 |

TECHNICAL FIELDS SEARCHED (Int.Cl. 3)

C 07 C 103/52
C 07 D 207/16
A 61 K 37/02

CATEGORY OF CITED DOCUMENTS

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons

&: member of the same patent family, corresponding document

The present search report has been drawn up for all claims

| Place of search | Date of completion of the search | Examiner |
|---|---|---|
| The Hague | 14-01-1982 | GRAMAGLIA |